# EUROPEAN PATENT APPLICATION

(11) **EP 2 090 884 A2**
(43) Date of publication of application: **19.08.2009**
(21) Application number: 09002009.0
(22) Date of filing: 13.02.2009
(51) Int. Cl.: G01N 27/416, G01N 33/487

(54) **Auto-calibrating test sensors**

(30) Priority: 15.02.2008 US 65873 P
(71) Applicant: Bayer HealthCare, LLC, Tarrytown, NY 10591 (US)
(72) Inventor: Sun, Hoi-Cheong S., Mount Kisco, New York 10549 (US); Johnson, Gary J., Langrangeville, NY 12540 (US); Holzer, Matthew, Bronx, NY 10463 (US); Wu, Mu, Granger, IN 46530 (US); Chen, Jun, Warren, New Jersey 07059 (US)
(74) Representative: Beyreuther, Stefan

(57) **Abstract**

A method of making a test sensor configured to assist in determining information related to an analyte in a fluid sample is disclosed. The method comprises the act of providing a base having a first end and a second opposing end. The method further comprises the act of providing a fluid-receiving area configured to receive a fluid sample. The method further comprises the act of assigning calibration information to the test sensor. The method further comprises the act of forming at least one notch such that a depth of the notch corresponds to the calibration information.

## Description

### FIELD OF THE INVENTION

The present invention relates generally to test sensors that are adapted to determine an analyte concentration. More specifically, the present invention generally relates to auto-calibrating test sensors.

### BACKGROUND OF THE INVENTION

The quantitative determination of analytes in body fluids is of great importance in the diagnoses and maintenance of certain physiological abnormalities. For example, lactate, cholesterol, and bilirubin should be monitored in certain individuals. In particular, it is important that diabetic individuals frequently check the glucose level in their body fluids to regulate the glucose intake in their diets. The results of such tests may be used to determine what, if any, insulin or other medication should be administered. In one type of blood-glucose testing system, test sensors are used to test a sample of blood.

A test sensor contains biosensing or reagent material that reacts with, for example, blood glucose. The testing end of the sensor is adapted to be placed into the fluid being tested (e.g., blood) that has accumulated on a person's finger after the finger has been pricked. The fluid may be drawn into a capillary channel that extends in the sensor from the testing end to the reagent material by capillary action so that a sufficient amount of fluid to be tested is drawn into the sensor. The tests are typically performed using optical or electrochemical testing methods.

Diagnostic systems, such as blood-glucose testing systems, typically calculate the actual glucose value based on a measured output and the known reactivity of the reagent-sensing element (e.g., test sensor) used to perform the test. The reactivity or lot-calibration information of the test sensor may be provided on a calibration circuit that is associated with the sensor package or the test sensor. This calibration circuit is typically physically inserted by the end user. In other cases, the calibration is automatically done using an auto-calibration circuit via a label on the sensor package or the test sensor. In this case, calibration is transparent to the end user and does not require that the end user insert a calibration circuit into the meter. Manufacturing millions of sensor packages, each having a calibration circuit or label to assist in calibrating the sensor package, can be expensive.

Therefore, it would be desirable to have a test sensor that provides calibration information thereon that may be manufactured in an efficient and/or cost-effective manner.

### SUMMARY OF THE INVENTION

According to one process, a method of making a test sensor configured to assist in determining information related to an analyte in a fluid sample is disclosed. The method comprises the act of providing a base having a first end and a second opposing end. The method further comprises the act of providing a fluid-receiving area configured to receive a fluid sample. The method further comprises the act of assigning calibration information to the test sensor. The method further comprises the act of forming at least one notch such that a depth of the notch corresponds to the calibration information.

According to another process, a method of making a test sensor configured to assist in determining information related to an analyte in a fluid sample is disclosed. The method comprises the act of providing a base having a first end and a second opposing end. The method further comprises the act of providing a fluid-receiving area configured to receive a fluid sample. The method further comprises the act of assigning calibration information to the test sensor. The test sensor includes at least one plate thereon such that the size of the at least one plate corresponds to the calibration information. The at least one plate also includes electrically-conductive material.

According to another process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one notch formed therein. The method further comprises the act of assigning calibration information to the test sensor. The method further comprises the act of providing a meter with a test-sensor opening. The meter includes a potentiometer positioned at or near the test-sensor opening. The potentiometer includes a movable actuator that receives the at least one notch. The method further comprises the act of placing the test sensor into the test-sensor opening of the meter. The method further comprises the act of moving the test sensor so as to move the movable actuator. The method further comprises the act of determining a measured resistance of the potentiometer. The method further comprises the act of applying the calibration information using the measured resistance to assist in determining the information related to the analyte in the fluid sample.

According to a further process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one notch formed therein. The method further comprises the act of assigning calibration information to the test sensor. The method further comprises the act of providing a meter with a test-sensor opening. The meter includes a pseudo potentiometer positioned at or near the test-sensor opening. The pseudo potentiometer includes a plurality of pads. The plurality of pads include resistive materials. The method further comprises the act of placing the test sensor into the test-sensor opening of the meter. The method further comprises the act of moving the test sensor so as to cause at least one of the plurality of pads to receive the at least one notch. The method further comprises the act of determining a measured resistance of the pseudo potentiometer. The method further comprises the act of applying the calibration information using the measured resistance to assist in determining the information related to the analyte in the fluid sample.

According to yet another process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one notch formed therein. The method further comprises the act of assigning calibration information to the test sensor. The method further comprises the act of providing a meter with a test-sensor opening. The meter includes a variable inductor positioned at or near the test-sensor opening. The variable inductor includes a movable plunger and at least one wire coil. The moveable plunger is configured to move within the at least one wire coil. The moveable plunger receives the at least one notch. The method further comprises the act of placing the test sensor into the test-sensor opening of the meter. The method further comprises the act of moving the test sensor so as to move the moveable plunger a distance within the at least one wire coil. The method further comprises the act of determining a measured electrical value of the variable inductor. The measured electrical value corresponds to the distance that the moveable plunger is moved within the at least one wire coil. The method further comprises the act of applying the calibration information using the measured electrical value to assist in determining the information related to the analyte in the fluid sample.

According to another process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one notch formed therein. The method further comprises the act of assigning calibration information to the test sensor. The method further comprises the act of providing a meter with a test-sensor opening. The meter includes a variable capacitor positioned at or near the test-sensor opening. The variable capacitor includes a movable plunger and a sleeve. The moveable plunger is configured to move within the sleeve. The moveable plunger also receives the at least one notch. The method further comprises the act of placing the test sensor into the test-sensor opening of the meter. The method further comprises the act of moving the test sensor so as to move the moveable plunger a distance within the sleeve. The method further comprises the act of determining a measured capacitance of the variable capacitor. The measured capacitance corresponding to the distance that the moveable plunger is pushed within the sleeve. The method further comprises the act of applying the calibration information using the measured capacitance to assist in determining the information related to the analyte in the fluid sample.

According to another process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one sensor plate thereon. The at least one sensor plate is made from electrically-conductive materials. The method further comprises the act of assigning calibration information to the test sensor. The method further comprises the act of providing a meter with a test-sensor opening. The meter includes a meter plate positioned at or near the test-sensor opening. The meter plate is made from electrically-conductive materials. The method further comprises the act of placing the test sensor into the test-sensor opening of the meter. The method further comprises the act of moving the test sensor such that at least a portion of the meter plate overlaps at least a portion of the at least one sensor plate. The method further comprises the act of determining a measured capacitance of the meter plate. The measured capacitance corresponds to the size of the at least one sensor plate. The method further comprises the act of applying the calibration information using the measured capacitance to assist in determining the information related to the analyte in the fluid sample.

According to another process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one sense portion. The method further comprises the act of assigning calibration information to the test sensor. The method further comprises the act of providing a meter with a test-sensor opening. The meter includes a parallel plate capacitor positioned at or near the test-sensor opening. The parallel plate capacitor includes two electrically-conductive plates. The parallel plate capacitor is configured to allow the at least one sense portion of the test sensor to be positioned between the two electrically-conductive plates. The method further comprises the act of placing the test sensor into the test-sensor opening of the meter. The method further comprises the act of moving the test sensor such that at least the sense portion of the test sensor is positioned between the two electrically-conductive plates. The method further comprises the act of determining a measured capacitance of the parallel plate capacitor. The method further comprises the act of applying the calibration information using the measured capacitance to assist in determining the information related to the analyte in the fluid sample.

According to another process, a method of using a test sensor and a meter is disclosed. The test sensor and meter use calibration information in determining information related to an analyte in a fluid sample. The method comprises the act of providing a test sensor including a base having a first end and a second opposing end. The test sensor further includes a fluid-receiving area configured to receive the fluid sample. The test sensor further includes at least one notch formed therein. The method further comprises the act of assigning calibration information to the test sensor. The method further comprises the act of providing a meter with a test-sensor opening. The meter includes a piezoelectric element positioned at or near the test-sensor opening. The piezoelectric element receives the at least one notch. The method further comprises the act of placing the test sensor into the test-sensor opening of the meter. The method further comprises the act of moving the test sensor so as to compress the piezoelectric element a distance. The method further comprises the act of determining a measured voltage of the piezoelectric element. The measured voltage corresponds to the distance that the piezoelectric element is compressed. The method further comprises the act of applying the calibration information using the measured voltage to assist in determining the information related to the analyte in the fluid sample.

The above summary is not intended to represent each embodiment or every aspect of the present invention. Additional features and benefits of the present invention are apparent from the detailed description and figures set forth below.

### BRIEF DESCRIPTION OF THE DRAWINGS

FIG. 1 a is a test sensor according to one embodiment.

FIG. 1b is a side view of the test sensor of FIG. 1a.

FIG. 2 is a cross-sectional view of a test sensor according to another embodiment.

FIG. 3 is an isometric view of an instrument or meter for receiving the test sensors of the embodiments of the present invention.

FIG. 4 is a top view of a test sensor being used with a plunger-type potentiometer according to another embodiment of the present invention.

FIG. 5 is a top view of the test sensor of FIG. 4 being used with a slide-type potentiometer according to another embodiment of the present invention.

FIG. 6 is a top view of a test sensor being used with a slide-type potentiometer according to another embodiment of the present invention.

FIG. 7 is a top view of a test sensor and a pseudo-potentiometer according to yet another embodiment of the present invention.

FIG. 8 is a top view of a test sensor being used with a plunger-type variable inductor according to one embodiment of the present invention.

FIG. 9 is a top view of the test sensor being used with a variable capacitor according to one embodiment of the present invention.

FIG. 10 is a top view of a test sensor being used with a variable capacitor according to another embodiment of the present invention.

FIG. 11 is a perspective view of a test sensor and a variable capacitor according to a further embodiment of the present invention.

FIG. 12 is a top view of a test sensor being used with an piezoelectric element according to one embodiment of the present invention.

While the invention is susceptible to various modifications and alternative forms, specific embodiments are shown by way of example in the drawings and are described in detail herein. It should be understood, however, that the invention is not intended to be limited to the particular forms disclosed. Rather, the invention is to cover all modifications, equivalents, and alternatives falling within the spirit and scope of the invention.

### DETAILED DESCRIPTION OF ILLUSTRATED EMBODIMENTS

Generally, an instrument or meter uses a test sensor adapted to receive a fluid sample to be analyzed and a processor adapted to perform a predefined test sequence for measuring a predefined parameter value. A memory is coupled to the processor for storing predefined parameter data values. Calibration information associated with the test sensor may be read by the processor before or after the fluid sample to be measured is received, but not after the analyte concentration has been determined. Calibration information is generally used to compensate for different characteristics of test sensors, which will vary on a batch-to-batch basis.

The calibration information may be, for example, the lot specific reagent calibration information for the test sensor. The calibration information may be in the form of a calibration code. Selected information associated with the test sensor (which may vary on a batch-to-batch basis) is tested to determine the calibration information to be used in association with the meter.

The present invention is directed to an improved method of making a test sensor that is adapted to assist in determining an analyte concentration. In one embodiment, a test sensor is adapted to receive a fluid sample. The fluid sample is analyzed using an instrument or meter. Analytes that may be measured include glucose, lipid profiles (e.g., cholesterol, triglycerides, LDL, and HDL), microalbumin, hemoglobin A_{1C}, fructose, lactate, or bilirubin. It is contemplated that other analyte concentrations may be determined. The analytes may be in, for example, a whole blood sample, a blood serum sample, a blood plasma sample, other body fluids like ISF (interstitial fluid), creatinine, urea, urine, and non-body fluids.

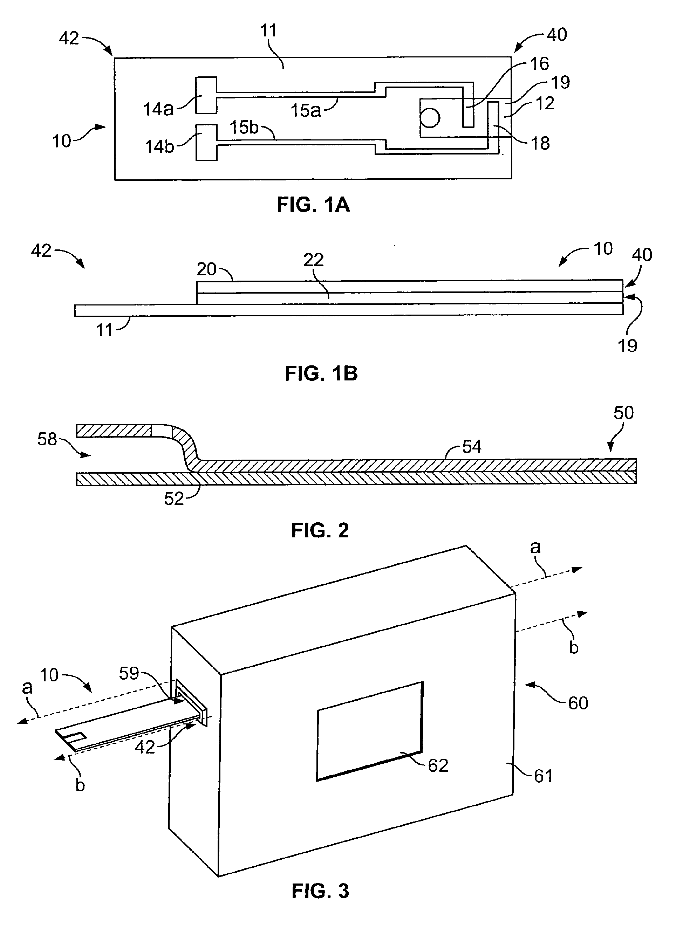

The test sensors described herein may be electrochemical test sensors. One non-limiting example of an electrochemical test sensor is shown in FIG. 1a. FIG. 1a depicts a test sensor 10 including a base 11, a capillary channel, and a plurality of electrodes 16 and 18. A region 12 shows an area that defines the capillary channel (e.g., after a lid is placed over the base 11). The plurality of electrodes includes a counter electrode 16 and a working (measuring) electrode 18. The electrochemical test sensor may also contain at least three electrodes, such as a working electrode, an auxiliary or "counter" electrode, a trigger electrode, or a hematocrit electrode. The electrodes 16, 18 are coupled to a plurality of conductive leads 15a,b, which, in the illustrated embodiment, terminate with a larger area designated as test-sensor contacts 14a,b. The capillary channel is generally located in a fluid-receiving area 19. It is contemplated that other electrochemical test sensors may be employed.

The fluid-receiving area 19 includes at least one reagent for converting the analyte of interest (e.g., glucose) in the fluid sample (e.g., blood) into a chemical species that is electrochemically measurable, in terms of the electrical current it produces, by the components of the electrode pattern. The reagent typically contains an enzyme such as, for example, glucose oxidase, which reacts with the analyte and with an electron acceptor such as a ferricyanide salt to produce an electrochemically measurable species that can be detected by the electrodes. It is contemplated that other enzymes may be used to react with glucose such as glucose dehydrogenase. If the concentration of another analyte is to be determined, an appropriate enzyme is selected to react with the analyte.

A fluid sample (e.g., blood) may be applied to the fluid-receiving area 19. The fluid sample reacts with the at least one reagent. After reacting with the reagent and in conjunction with the plurality of electrodes, the fluid sample produces electrical signals that assist in determining the analyte concentration. The conductive leads 15a,b carry the electrical signal back toward a second opposing end 42 of the test sensor 10 where the test-sensor contacts 14a,b transfer the electrical signals into the meter.

Referring to FIG. 1b, a side view of the test sensor 10 of FIG. 1a is shown. As shown in FIG. 1b, the test sensor 10 of FIG. 1b further includes a lid 20 and a spacer 22. The base 11, the lid 20, and the spacer 22 may be made from a variety of materials such as polymeric materials. Non-limiting examples of polymeric materials that may be used to form the base 11, the lid 20, and the spacer 22 include polycarbonate, polyethylene terephthalate (PET), polyethylene naphthalate (PEN), polyimide, and combinations thereof. It is contemplated that other materials may be used in forming the base 11, lid 20, and/or spacer 22.

To form the test sensor 10 of FIGs. 1a, 1b, the base 11, the spacer 22, and the lid 20 are attached by, for example, an adhesive or heat sealing. When the base 11, the lid 20, and the spacer 22 are attached, a fluid-receiving area 19 is formed. The fluid-receiving area 19 provides a flow path for introducing the fluid sample into the test sensor 10. The fluid-receiving area 19 is formed at a first end or testing end 40 of the test sensor 10.

It is contemplated that the test sensors of the embodiments of the present invention may be formed with a base and a lid in the absence of a spacer. In one such embodiment, a lid may be formed with a convex opening that is adapted to receive a fluid. A non-limiting example of such a test sensor is shown in FIG. 2. Specifically, in FIG. 2, a test sensor 50 includes a base 52 and a lid 54. When the lid 54 is attached to the base 52, a fluid-receiving area 58 is formed that is adapted to receive fluid for testing.

The test sensors of the embodiments described herein may be optical test sensors. Optical test-sensor systems may use techniques such as, for example, transmission spectroscopy, diffuse reflectance, or fluorescence spectroscopy for measuring the analyte concentration. An indicator reagent system and an analyte in a sample of body fluid are reacted to produce a chromatic reaction, as the reaction between the reagent and analyte causes the sample to change color. The degree of color change is indicative of the analyte concentration in the body fluid. The color change of the sample is evaluated to measure the absorbance level of the transmitted light.

Referring back to FIGs. 1a,b, the second opposing end 42 of the test sensor 10 is adapted to be placed into a test-sensor opening 59 in an instrument or meter 60, as shown, for example, in FIG. 3. FIG. 3 depicts a single-sensor instrument or meter 60. The meter 60 includes a housing 61 that forms the test-sensor opening 59, which is of sufficient size to receive the second opposing end 42 of the test sensor 10. After the calibration information of the test sensor 10 has been determined, the meter 60 uses, for example, the appropriate program number. The meter housing 61 may include a display 62 (e.g., an LCD screen) that displays, for example, analyte concentrations.

According to one embodiment of the present invention, a test-sensor combination may be used with at least one potentiometer to determine calibration information corresponding with a particular test sensor. The potentiometer is generally housed within a meter (e.g., meter 60 of FIG. 3) and in one embodiment is located on a printed circuit board within and near the back or inner portion of the test-sensor opening (e.g., opening 59 of FIG. 3) formed in the housing of a meter. The potentiometer may include a moveable actuator that is moved to set a measured resistance. The test sensors of these embodiments form one or more apertures or notches. The one or more notches may receive and move the actuator when the test sensor is inserted into the test-sensor opening of a meter.

When the test sensor is inserted through the test-sensor opening, a notch formed in the test sensor contacts the actuator and pushes the actuator, thereby setting the measured resistance. The depth of the notch(es) determines the distance that the actuator moves, which corresponds to the measured resistance. The measured resistance is compared to stored nominal resistances corresponding with particular types of calibration information. Each nominal resistance associated with each different type of test sensor includes an acceptable boundary or deviation. The acceptable boundary is determined based upon the accuracy of the potentiometer. The calibration information having an associated nominal resistance closest to the measured resistance and within the acceptable boundary of the nominal resistance is applied.

The depth of the notch is, thus, varied among test sensors having different calibration information. Test sensors having notches of different depths push the actuator different distances. More specifically, test sensors having notches of greater depths push the actuator shorter distances, and test sensors having notches of smaller depths push the actuator larger distances.

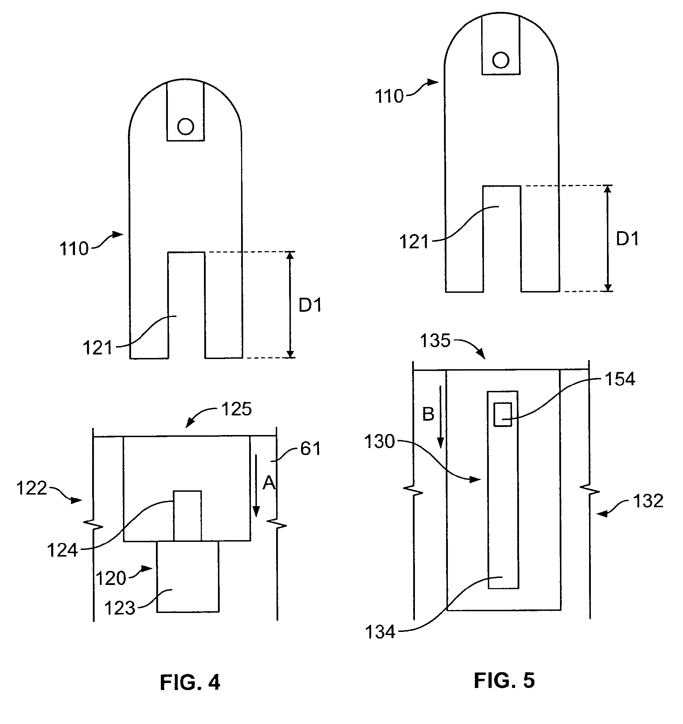

FIG. 4 illustrates a test sensor 110 being used with a plunger-type potentiometer 120 according to one embodiment. FIG. 4 shows a view of a meter 122 generally taken through a plane (e.g., a plane formed between from line a-a and line b-b of FIG. 3) running through a portion (e.g., the test-sensor opening) of the length of the meter 122. The test sensor 110 includes an aperture or notch 121 formed through at least one of a base, spacer, or lid of the test sensor 110. The notch 121 has a depth, D1, associated therewith. As discussed above, the test sensors with different calibration information will have varying depths. The plunger-type potentiometer 120 is positioned within and near the back or inner portion of a test-sensor opening 125. The plunger-type potentiometer 120 includes a body 123 and an actuator 124 that extends in the direction of the opening 125. The actuator 124 is adapted to be pushed toward the body 123 of the potentiometer 120 (e.g. in the direction of Arrow A) when the test sensor 110 is inserted into the opening 125. The distance that the actuator 124 is pushed sets a measured resistance. The measured resistance is compared to a plurality of stored nominal resistances, which are associated with a plurality of types of calibration information. The calibration information having an associated nominal resistance (within an acceptable boundary) closest to the measured resistance is applied.

FIG. 5 illustrates the test sensor 110 of FIG. 4 being used with a slide-type potentiometer 130 according to another embodiment of the present invention. It is contemplated that test sensors other than the test sensor 110 of FIG. 4 may be used with the slide-type potentiometer 130. FIG. 5 illustrates a view of a meter 132 generally taken through a plane (e.g., a plane formed between from line a-a and line b-b of FIG. 3) running through a portion (e.g., the test-sensor opening) of the length of the meter 132. The slide-type potentiometer 130 of FIG. 5 is mounted on a bottom, interior surface of the meter 132 within and near the back or inner portion of a test-sensor opening 135. The slide-type potentiometer 130 may also be mounted on a top, interior surface of the meter 132. The slide-type potentiometer 130 includes a body 134 and an actuator 154 extending in a generally upward direction. In other embodiments, the actuator 154 extends in a generally downward direction. The actuator 154 is adapted to be pushed in the direction of Arrow B when the test sensor 160 is inserted into the opening 135. The distance that the actuator 154 is pushed sets a measured resistance. The measured resistance is compared to a plurality of stored nominal resistances, which are associated with a plurality of types of calibration information. The calibration information having an associated nominal resistance (within an acceptable boundary) closest to the measured resistance is applied.

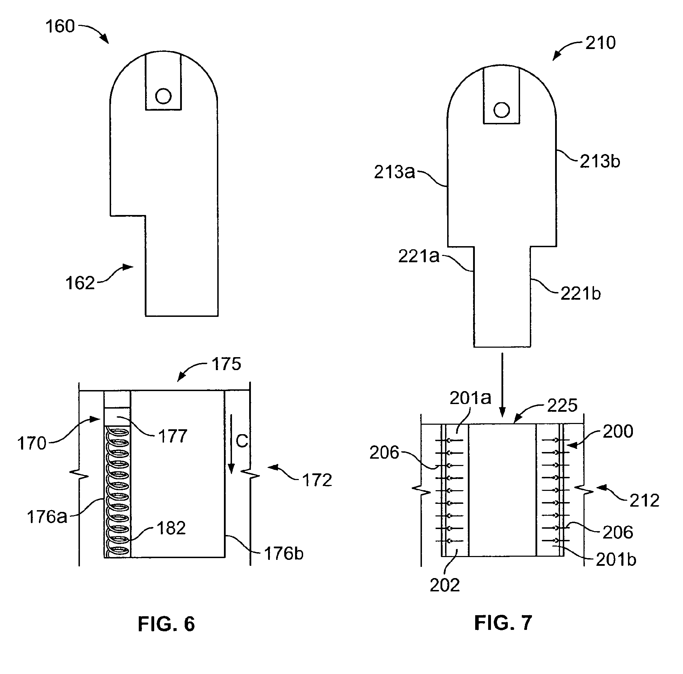

FIG. 6 shows a test sensor 160 being used with a slide-type potentiometer 170 according to another embodiment. The test sensor 160 includes an aperture or notch 162 formed through at least one of a base, spacer, or lid of the test sensor 160. FIG. 6 illustrates a view of a meter 172 generally taken through a plane (e.g., a plane formed between from line a-a and line b-b of FIG. 3) running through a portion (e.g., the test-sensor opening) of the length of the meter 172. The slide-type potentiometer 170 of FIG. 6 is mounted on a side 176a of a test-sensor opening 175 formed on the housing of the meter 172. The slide-type potentiometer 170 may also be mounted on a second opposing side 176b of the test-sensor opening 175 of the meter 172. It is contemplated that in some embodiments a slide-type potentiometer 170 may be mounted on both side 176a and side 176b. The slide-type potentiometer 170 includes an actuator 177 that extends from the side 176a of the opening 59. The actuator 177 is adapted to be pushed in the direction of Arrow C when the test sensor 110 is inserted into the opening 175. The distance that the actuator 177 is pushed sets a measured resistance. The measured resistance is compared to a plurality of stored nominal resistances, which are associated with a plurality of types of calibration information. The calibration information having an associated nominal resistance (within an acceptable boundary) closest to the measured resistance is applied.

After the test sensors illustrated and described above in FIGs. 4-6 are removed from the test-sensor opening, a spring mechanism may be used to return the actuators to their default settings (e.g., highest or lowest resistance). FIG. 6 illustrates one non-limiting example of a spring mechanism (spring mechanism 182). Referring to FIG. 6, for example, a mechanism may be provided within the test-sensor opening 172 to retain the test sensor 160 in a fully inserted position against the outward force being applied by the spring mechanism 182. The meters may further include a retaining device and/or a sensor-release button.

FIG. 7 illustrates a pseudo-potentiometer 200 and a test sensor 210 adapted to be used therewith, according to another embodiment of the present invention. FIG. 7 shows a view of a meter 212 generally taken through a plane (e.g., a plane formed between from line a-a and line b-b of FIG. 3) running through a portion (e.g., the test-sensor opening) of the meter 212. The test sensor 210 includes a pair of notches 221a,b formed along opposing sides 213a,b of the test sensor 210. The pseudo-potentiometer 200 is formed using pads of resistive material 201a,b positioned on a printed circuit board 202 within a test-sensor opening 225 formed on the meter 212. The pads 201a,b include a plurality of contacts 206. After the test sensor 210 has been inserted into the opening 225, the contacts 206 contact the pads 201a,b where the sensor 210 is notched. Where the sensor is unnotched, however, the unnotched portion of the test sensor 210 becomes positioned between the pads 201 a,b and the contacts 206, thereby breaking contact between the pads 201a,b and the contacts 206. The measured resistance of the pseudo-potentiometer 200 is determined by the number of contacts 206 and/or the position of the contacts 206 contacting the pads 201 a,b, which is determined by the depth of the notches 221 a,b. The measured resistance is compared to a plurality of stored nominal resistances, which are associated with a plurality of types of calibration information. The calibration information having an associated nominal resistance (within an acceptable boundary) closest to the measured resistance is applied.

The embodiment of FIG. 7 may be desirable to eliminate the need for a spring mechanism (e.g., spring mechanism 182 of FIG. 6), retaining device, sensor-release button, or the like, as described above with respect to FIGs. 4-6. Although in the embodiment of FIG. 7, two pads of resistive material 201a,b and two notches 221a,b are shown, it is contemplated that a different number (e.g., one) of pads and/or notches may be used. Furthermore, any number of contacts 206 may be used.

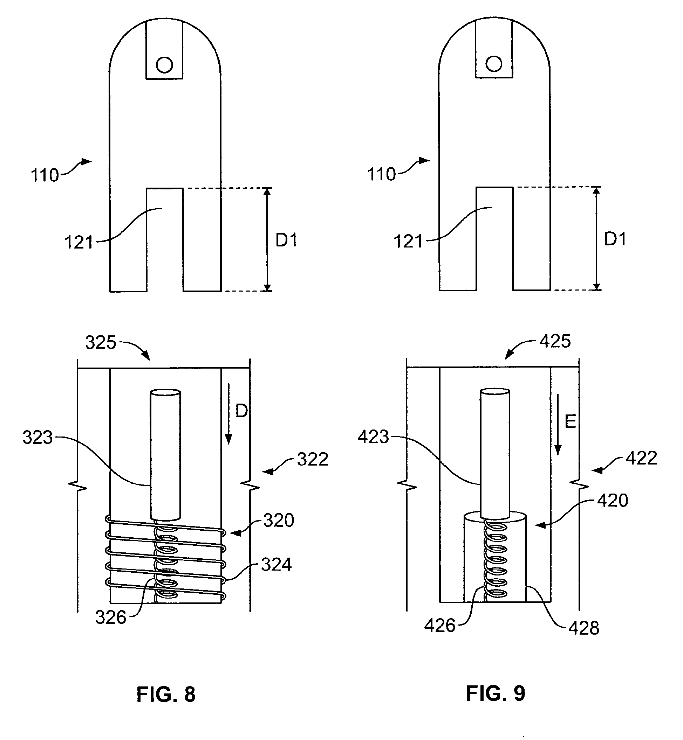

According to other embodiments of the present invention, a test-sensor combination may be used with a variable inductor to determine calibration information corresponding with a particular test sensor. FIG. 8 illustrates a test sensor 110 being used with a variable inductor 320 according to one embodiment. FIG. 8 shows a view of a meter 322 generally taken through a plane (e.g., a plane formed between from line a-a and line b-b of FIG. 3) running through a portion (e.g., the test sensor opening) of the length of the meter 322. The variable inductor 320 is generally housed within a meter 322 and is positioned within and near the back or inner portion of a test-sensor opening 325 formed in the housing of the meter 322. The variable inductor 320 is formed from a moveable plunger 323 and one or more wire coil(s) 324 wound in at least one turn around a back portion of the test-sensor opening 325. It is contemplated that the wire coil(s) 324 may be shaped in various configurations including, but not limited to, flat coils. It is further contemplated that the moveable plunger 323 may be shaped in various configurations including, but not limited to, a flat member, a circular member, or any generally rod-like structure. The plunger 323 is adapted to move in the direction of Arrow D when the test sensor 110 is inserted into the test-sensor opening 325. The plunger 323 is initially positioned such that it partially or completely extends beyond the wire coil(s) 324 when no test sensor 110 is inserted within the test-sensor opening 325 of the meter 322. A spring mechanism 326 may be used to return the plunger 323 to its initial position when a test sensor 110 is removed from the test-sensor opening 325. As described above, the test sensor 110 forms an aperture or notch 121 through at least one of a base, spacer, or lid of the test sensor 110. The one or more notches 121 may receive and move the plunger 323 when the test sensor 110 is inserted into the test-sensor opening 325 of the meter 322. It is contemplated that suitable test sensors other than the test sensor 110 shown in FIG. 8 may be used with the variable inductor 320.

When the test sensor 110 is inserted through the test-sensor opening 325, the notch 121 contacts the plunger 323 and pushes the plunger 323 a distance within the wire coil(s) 324, thereby changing the permeability of the core of the variable inductor 320. As the permeability of the core increases, the inductance of the wire coil(s) 324 increases. Likewise, as the permeability of the core decreases, the inductance of the wire coil(s) 324 decreases. Thus, the distance that the plunger 323 is pushed determines the inductance of the wire coil(s) 324. It is contemplated that the wire coil(s) 324 may be positioned within the test-sensor opening 325 such that the plunger 323 is pushed into or out of the wire coil(s) when the test sensor 110 is inserted through the test-sensor opening 325. It is also contemplated that the plunger 323 may be made from materials having high magnetic susceptibility (e.g., ferromagnetic materials) to achieve greater changes in inductance relative to the plunger's 323 position within the wire coil(s) 324.

The inductance may be directly measured by, for example, a frequency counter or an AC bridge. The measured inductance value is compared to stored inductances values corresponding with particular types of calibration information. Each stored inductance value associated with each different type of test sensor 110 includes an acceptable boundary or deviation. The acceptable boundary is based upon the accuracy of the variable inductor system 320. The calibration information having an associated stored inductance value closest to the measured inductance value and within the acceptable boundary of the stored inductance value is applied. It is contemplated that in some embodiments other electrical characteristics directly affected by the inductance of the wire coil(s) including, but not limited to, voltage, current, resonant frequency, reactance, or combinations thereof may be measured and compared.

The depth of the notch 121 is, thus, varied among test sensors 110 having different calibration information. As described above, test sensors having notches of different depths push the plunger 323 different distances. More specifically, test sensors having notches of greater depths push the plunger 323 shorter distances, and test sensors having notches of smaller depths push the plunger 323 larger distances.

In one aspect, a linear variable differential transformer (hereinafter "LVDT") is formed from the plunger 323 and the wire coil(s) 324. Any suitable additional circuitry necessary to complete the LVDT circuit may be implemented on a microprocessor or a printed circuit board. There are several advantages associated with LVDT configurations. For example, LVDT circuits have minimal, if any, sensitivity to movements of the plunger 323 in any direction other than the direction along the cental axis of the wire coil(s) 324 (e.g., parallel to Arrow D). More importantly, the distance that the plunger 323 is pushed within the wire coil(s) 324 is linearly related to the output voltage of the LVDT circuit over a determined range of distances depending on the specific LVDT circuit designed. The output voltage of the LVDT circuit is measured and compared to a plurality of stored voltage values, which are associated with a plurality of types of calibration information. The calibration information having an associated stored voltage value (within an acceptable boundary) closest to the measured voltage value is applied. It is contemplated that the LVDT circuit may be implemented either in analog or digital. However, utilizing digital LVDT circuits may be more cost effective.

In some variable inductor embodiments, the plunger 323 may be made from electrically conductive material(s) such as, for example, silicon carbide, graphite, silver, gold, platinum, other metals, metal alloys, or combinations thereof. According to these embodiments, an LC circuit is formed by the wire coil(s) 324 and the plunger 323. As previously described, the distance that the plunger 323 is pushed determines the inductance of the wire coil(s) 324. The change in inductance of the wire coil(s) 324 correspondingly alters the resonant frequency of the LC circuit. The resonant frequency may be measured by, for example, coupling to a probe coil, a frequency counter, or an AC bridge. The measured resonant frequency value is compared to a plurality of stored resonant frequency values, which are associated with a plurality of types of calibration information. The calibration information having an associated stored resonant frequency value (within an acceptable boundary) closest to the measured resonant frequency value is applied. Alternatively, a base-line resonant frequency may be established for the LC circuit when no test sensor 110 is inserted within the test-sensor opening 325. When a test sensor 110 is inserted into the test-sensor opening 325, deviations of the resonant frequency from the base-line resonant frequency are measured and compared to a plurality of stored resonant frequency deviations as described above. In other variable inductor LC circuit embodiments, the reactance of the LC circuit may be measured by, for example, a frequency counter or an AC bridge. The measured reactance value is compared to a plurality of stored reactance values as described above.

According to other embodiments of the present invention, a test-sensor combination may be used with a variable capacitor to determine calibration information corresponding with a particular test sensor. The variable capacitor may be comprised of components housed entirely within a meter or, alternatively, a portion of the variable capacitor may be included on a test sensor. Regardless of the location of the components of the variable capacitor, the capacitance of the variable capacitor may be varied by increasing or decreasing the surface area of the components of the variable capacitor. For example, the variable capacitor may be a parallel plate capacitor with differently sized plates. Assuming the distance separating the plates remains constant, increasing the size (i.e., surface area) of the smaller plate, increases the surface area of the parallel plate capacitor, thus, directly increasing the capacitance of the parallel plate capacitor. Another type of variable capacitor may be a set of concentric cylinders where one cylinder can be slid in or out of an opposing cylinder. As the inner cylinder is slid further into the outer cylinder, the surface area and, thus, capacitance of the variable capacitor increases. Likewise, as the inner cylinder is moved out of the outer cylinder, the capacitance decreases. Concentric cylinder variable capacitors may be desirable because the distance between the cylinders remains constant. Alternatively, the capacitance of the variable capacitor may be varied by changing the type of dielectric material that is between the capacitor plates, cylinders, or components.

The test sensors are generally configured to interact with the variable capacitor such that the measured capacitance of the variable capacitor directly depends upon characteristics of the particular test sensor inserted. The measured capacitance is compared to stored capacitances corresponding with particular types of calibration information. Each stored capacitance associated with each different type of test sensor includes an acceptable boundary or deviation. The acceptable boundary is based upon the accuracy of the variable capacitor system. The calibration information having an associated stored capacitance closest to the measured capacitance and within the acceptable boundary of the stored capacitance is applied.

FIG. 9 illustrates a test sensor 110 being used with a variable capacitor 420 according to one embodiment. FIG. 9 shows a view of a meter 422 generally taken through a plane (e.g., a plane formed between from line a-a and line b-b of FIG. 3) running through a portion (e.g., the test sensor opening) of the length of the meter 422. As described above, the test sensor 110 includes an aperture or notch 121 formed through at least one of a base, spacer, or lid of the test sensor 110. It is contemplated that suitable test sensors other than the test sensor 110 may be used with the variable capacitor 420. For example, the test sensors used with the variable capacitor 420 may include more than one notch 121. The variable capacitor 420 is positioned within and near the back or inner portion of a test-sensor opening 425.

The variable capacitor 420 illustrated in FIG. 9 includes a set of concentric cylinders where an inner cylinder is a conductive plunger 423 that can be moved in or out of an outer cylinder, which is a sleeve 428. The sleeve 424 may be, for example, a polymeric tube. The plunger 423 is initially positioned such that it partially or completely extends beyond the sleeve 428 when no test sensor 110 is inserted within the test-sensor opening 425 of the meter 422. The plunger 423 is adapted to be pushed in the direction of Arrow E into the sleeve 428 when a test sensor 110 is inserted into the test-sensor opening 425. A spring mechanism 426 or other suitable mechanism may be used to return the plunger 423 to its initial position when the test sensor 110 is removed from the test-sensor opening 425. It is contemplated that the plunger 423 may be sized and supported such that it does not contact the sleeve 428 as it moves within the sleeve 428. This may be beneficial because non-contacting concentric cylinders have less frictional forces acting upon them, thereby improving the service life of the variable capacitor 420. Alternatively, it is contemplated that a suitable dielectric material may be permanently attached to either the plunger 423 and/or the sleeve 428 to facilitate the movement and support of the plunger 423 within the sleeve 428. According to some embodiments, a dielectric material is attached to either the exterior surface of the plunger 423 or the interior surface of the sleeve 428 and a thin layer of material such as, for example, Teflon is attached to the dielectric material to better facilitate movement of the plunger 423 within the sleeve 428.

When the test sensor 110 is inserted through the test-sensor opening 425, the one or more notches 121 in the test sensors 110 receive and push the plunger 423 a distance within the sleeve 428, thereby varying the capacitance of the variable capacitor 420. The depth of the notch 121 determines the distance that the plunger 423 moves, which corresponds to the capacitance of the variable capacitor 420. The capacitance may be directly measured by, for example, a frequency counter or an AC bridge. The measured capacitance value is compared to stored capacitance values corresponding with particular types of calibration information. The calibration information having an associated stored capacitance value (within an acceptable boundary) closest to the measured capacitance value and within the acceptable boundary of the stored capacitance is applied.

The depth of the notch 121 is, thus, varied among test sensors 110 having different calibration information. As previously described, test sensors having notches of different depths push the plunger 423 different distances into the sleeve 428. More specifically, test sensors having notches of greater depths push the plunger 423 shorter distances, and test sensors having notches of smaller depths push the plunger 423 larger distances.

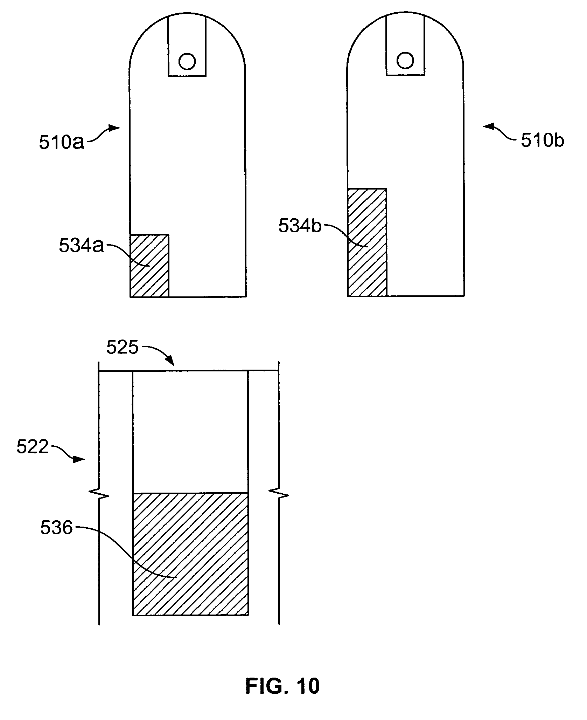

FIG. 10 illustrates a test sensor 510a being used with a variable capacitor according to another embodiment. FIG. 10 shows a view of a meter 522 generally taken through a plane (e.g., a plane formed between from line a-a and line b-b of FIG. 3) running through a portion (e.g., the test-sensor opening) of the length of the meter 522. The variable capacitor may include a pair of conductive surfaces 534a,536. A first conductive surface, sensor plate 534a, is mounted on a portion of a test sensor 510a. A second conductive surface, meter plate 536, is mounted on a top, interior surface of the meter 522 within and near the inner portion of a test-sensor opening 525. The meter plate 536 may alternatively be mounted on a bottom, interior surface of the meter 522. The meter plate 536 is positioned within the test-sensor opening 525 such that when the test sensor 510a is inserted through the test-sensor opening 525 of the meter 522, at least a portion of the meter plate 536 overlaps at least a portion of the sensor plate 534a. Likewise, the sensor plate 534a may be mounted anywhere on the test sensor 510a such that at least a portion of the sensor plate 534a overlaps at least a portion of the meter plate 536 when the test sensor 510a is inserted within the test-sensor opening 525. When the meter plate 536 and the sensor plate 534a (or portions thereof) overlap, the meter plate 536 and the sensor plate 534a are separated by a gap. It is contemplated that the conductive surfaces 534a,536 may have various shapes and sizes including, but not limited to, flat rectangular surfaces.

Without a test sensor 510a inserted through the test-sensor opening 525, the variable capacitor has negligible, if any, measured capacitance because there is no parallel plate capacitor formed by the sensor plate 534a and meter plate 536. Upon inserting a test sensor 510a through the test-sensor opening 525, at least a portion of the sensor plate 534a overlaps with the meter plate 536, thereby forming a parallel plate variable capacitor across the two conductive surfaces. The surface area of the overlapping portions of the sensor plate 534a and the meter plate 536 determines the surface area of the variable capacitor, which directly corresponds to the capacitance of the variable capacitor.

The capacitance may be measured by, for example, a frequency counter or an AC bridge. The measured capacitance value is compared to stored capacitance values corresponding with particular types of calibration information. The calibration information having an associated stored capacitance value (within an acceptable boundary) closest to the measured capacitance value is applied.

The size of the sensor plate is, thus, smaller than the meter plate 536 and is varied among test sensors having different calibration information. It is contemplated that the size of the sensor plate 534a may be varied according to positioning, width, length, or any combinations thereof as shown, for example, by a test sensor 510b having sensor plate 534b. Varying the size of the sensor plate, varies surface areas for the variable capacitor. More specifically, test sensors having sensor plates of greater size cause the variable capacitor to have a larger surface area and, thus, larger measured capacitance values. Likewise, test sensors having sensor plates of smaller sizes cause the variable capacitor to have a smaller surface area and, thus, smaller measured capacitance values. It is contemplated that the size of the sensor plate may be fabricated by, for example, laser trimming or other suitable methods.

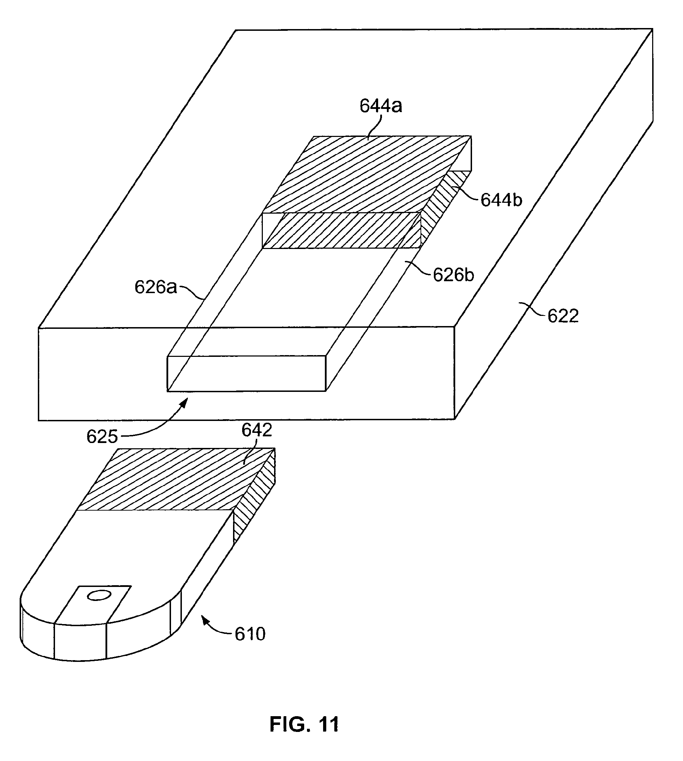

FIG. 11 illustrates a test sensor 610 being used with a variable capacitor according to another embodiment. FIG. 11 shows a view of a test-sensor opening 625 in a meter 622. The variable capacitor illustrated in FIG. 11 is a parallel plate capacitor including two electrode plates 644a,b. The electrode plates 644a,b are respectively mounted on top and bottom interior surfaces of the meter 622 within and near the inner portion of the test-sensor opening 625. It is contemplated that the electrodes plates 644a,b may alternatively be positioned on the left and right sides 626a,626b of the meter 622 within the test-sensor opening 625.

When no test sensor 610 is positioned within the test-sensor opening 625, the dielectric between the electrode plates 644a,b is air. However, when a test sensor 610 is inserted through the test-sensor opening 625, a sensor portion 642 of the test sensor 610 is positioned between the electrode plates 644a,b and, thus, the dielectric between the electrode plates 644a,b is a product of the physical characteristics of the sensor portion 642. Physical characteristics of the sensor portion 642 that affect the dielectric include, but are not limited to, material type, thickness, size, combinations thereof, or the like.

When the dielectric between electrode plates 644a,b changes, the capacitance of the variable capacitor changes correspondingly. As a result, when test sensors having sensor portions 642 of different dielectric characteristics are inserted into the meter 622, different capacitances are measured for the variable capacitor. The characteristics of the sensor portion 642 between the electrode plates 644a,b are, thus, varied among test sensors 610 having different calibration information. For example, test sensors having different calibration information associated therewith may include sensor portions 642 having different sizes, thicknesses, materials, combinations thereof, or the like.

The capacitance values of the variable capacitor are measured by, for example, a frequency counter or an AC bridge. The measured capacitance value is compared to stored capacitance values corresponding with particular types of calibration information. The calibration information having an associated stored capacitance value (within an acceptable boundary) closest to the measured capacitance value is applied. It is contemplated that instead of or in addition to capacitance, the frequency of the variable capacitor may be measured and compared, as previously described, to determine the associated calibration information for a particular test sensor 610.

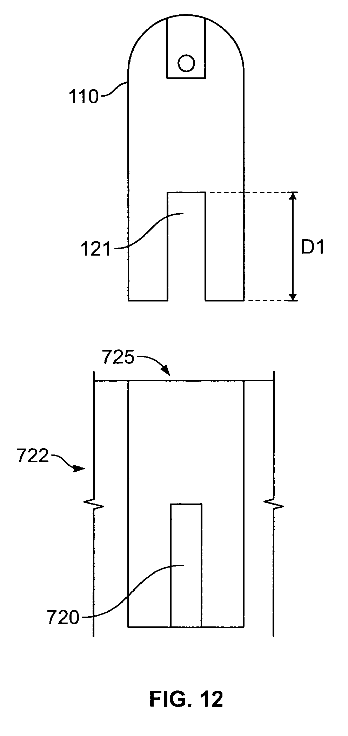

According to other embodiments of the present invention, a test-sensor combination may be used with one or more piezoelectric elements to determine calibration information corresponding to a particular test sensor. FIG. 12 illustrates a test sensor 110 being used with a piezoelectric element 720 according to one embodiment. FIG. 12 shows a view of a meter 722 generally taken through a plane (e.g., a plane formed between from line a-a and line b-b of FIG. 3) running through a portion (e.g., the test-sensor opening) of the length of the meter 722. The piezoelectric element 720 is positioned within and near the back or inner portion of a test-sensor opening 725 of the meter 722. Non-limiting examples of material suitable to form the piezoelectric element 720 include, quartz or barium titanate. As described above, the test sensors 110 form one or more apertures or notches 121. The one or more notches 121 may receive and compress the piezoelectric element 720 when the test sensor 110 is inserted through the test-sensor opening 725 of the meter 722.

When the test sensor 110 is inserted through the test-sensor opening 725, the notch 121 contacts the piezoelectric element 720 and compresses the piezoelectric element 720, thereby generating an output voltage on the piezoelectric element 720. The depth of the notch 121 determines the amount of compression, which corresponds to the measured voltage. The measured voltage is compared to stored voltages corresponding with particular types of calibration information. Each stored voltage associated with each different type of test sensor includes an acceptable boundary or deviation. The acceptable boundary is determined based upon the accuracy of the piezoelectric element 720. The calibration information having an associated stored voltage closest to the measured voltage and within the acceptable boundary of the stored voltage is applied.

The depth of the notch(es) are, thus, varied among test sensors having different calibration information. Test sensors having notches of different depths compress the piezoelectric element 720 different distances. More specifically, test sensors having notches of greater depths compress the piezoelectric element 720 shorter distances, and test sensors having notches of smaller depths compress the piezoelectric element 720 larger distances.

Any of the test sensors described above with respect to FIGs. 4-9 and 12 may be used with any of the embodiments described herein, provided that the actuator, plunger, or piezoelectric element are aligned with the notch or notches of the test sensors. It is contemplated that a meter may employ a combination of any of the embodiments described with respect to FIGS. 4-12 to determine the calibration information associated with a particular test sensor. It is also contemplated that meters other than the meter 60 illustrated in FIG. 3 may be used. Moreover, it is contemplated that the test sensors described with respect to FIGs. 4-9 and 12 may include any number of notches. When more than one notch is included, an additional corresponding actuator, plunger, or piezoelectric element is typically provided to be received by each additional notch. Increasing the number of notches increases the amount of distinct types of calibration information that may be distinguished by the meter. For example, assuming that a series of test sensors having one notch provides for X distinct types of calibration information, two notches would provide for X² different types of calibration information. The notches may be formed by methods such as punching or laser-cutting.

In one non-limiting embodiment, a maximum notch-depth corresponds with a default position of the actuator of FIGS. 4-7, and a zero notch-depth (i.e., a sensor not having a notch) corresponds to a fully displaced actuator. Thus, the range of available notch-depths corresponds with a full range of resistance of the potentiometer. In the case of a linear potentiometer, for example, a constant, linear relationship exists between resistance and notch depth. Similar notch-depth to measured electrical value (e.g., resistance, inductance, capacitance, frequency, voltage, etc.) relationships may be designed for the variable inductor, variable capacitor, and piezoelectric embodiments described herein.

The accuracy and sensitivity of devices such as the potentiometer, variable inductor, variable capacitor, or piezoelectric element must be considered when determining how many notch depths and, accordingly, how many different types of calibration information may be ascertained by the devices. More accurate and sensitive devices may detect and distinguish more minor variations in the measured values and notch depths. Thus, more accurate devices may be used to distinguish a series of test sensors having a larger number of notch-depth variations.

To provide error checking for each of the test sensors described with respect to FIGs. 4-12, the measured electrical value (e.g., resistance, inductance, capacitance, frequency, voltage, etc.) may be compared to the stored electrical values corresponding to the various types of calibration information. If the measured electrical value falls outside of the acceptable boundary on either side of the stored value associated with a type of calibration information, an invalid sensor error condition will be reported. Such error checking may prevent a defective sensor from being used. A persistent error condition may indicate that the potentiometer, variable inductor, variable capacitor, piezoelectric element or meter is defective or damaged.

To provide additional error checking for the meter, the resistance of the potentiometer, the inductance of the variable inductor, the capacitance of the variable capacitor, or output voltage of the piezoelectric element may be measured when no test sensor is present. This resistance, inductance, capacitance, or output voltage may then be compared to the expected default value. Such error checking may be used to detect, for example, a defective potentiometer, e.g., one in which the actuator is stuck, has a broken spring mechanism, or the like.

Additionally, because the operating characteristics of each individual potentiometer, variable inductor, variable capacitor, or piezoelectric element may vary from device to device, the meter used with the assemblies described with respect to FIGs. 4-12 may be calibrated at the time of manufacture. The meter calibration depends on exact operating characteristics and device variability. In an extreme case using a non-linear potentiometer, variable inductor, variable capacitor, or piezoelectric element having large variations in the operating ranges among devices, it may be necessary to measure and store the expected resistance, inductance, capacitance, or output voltage for each available type of calibration information using a standard set of special test sensors. Depending upon the wear characteristics of the potentiometer, variable inductor, variable capacitor, or piezoelectric element, it may also be possible to recalibrate periodically by re-measuring the default resistance, inductance, capacitance, or output voltage and adjusting the stored values associated with the different types of calibration information accordingly.

Assemblies such as those illustrated in FIGs. 4-12 may be desirable for several reasons. For example, the meters do not require any additional electrical contacts between the printed circuit board and the test sensor, other than the contacts required to activate and take readings from the test sensor. Furthermore, the assemblies do not require additional printing material on the sensor or, in some embodiments, laser cutting of conductive materials. Thus, the assemblies require a generally simpler, less expensive manufacturing process compared to existing assemblies.

All of the sensors and assemblies described herein may be desirable because they may support many different types of calibration information. The test sensors may be used as single stand-alone test sensors. The test sensors of the embodiments described herein may also be stored in a cartridge.

In the embodiments described herein, it is important that the test sensors are fully inserted into the test-sensor opening for the calibration information to be correctly ascertained. Thus, the meters used with the test sensors may include a mechanism for determining whether the test sensors are fully inserted. The mechanism may be positioned, for example, in or adjacent to the test-sensor opening. The meter may further be adapted to report an error to a user if it detects that the test sensor is not fully inserted.

After the test sensors illustrated and described above in FIGs. 4-12 are removed from the test-sensor opening, a spring mechanism may be used to return the potentiometers, variable inductors, or variable capacitors to their default settings. In those embodiments, a retaining mechanism may be provided within the test-sensor opening that is adapted to retain the test sensor in a fully inserted position against the outward force being applied by the spring mechanism. The meters may further include a retaining device and/or a sensor-release button.

The calibration information referred to herein may be any information that may be used by a meter or instrument. For example, the calibration information may be a program auto-calibration number that relates to a slope and intercept of calibration lines for the test sensor lot or batch. In addition to calibration information, other information may be contained such an analyte type or manufacturing date.

### PROCESS A

A method of making a test sensor configured to assist in determining information related to an analyte in a fluid sample, the method comprising the acts of:
providing a base having a first end and a second opposing end;
providing a fluid-receiving area configured to receive a fluid sample;
assigning calibration information to the test sensor; and
forming at least one notch such that a depth of the notch corresponds to the calibration information.

### PROCESS B

The method of Process A, wherein the at least one notch is formed at or near the second opposing end of the base.

### PROCESS C

A method of making a test sensor configured to assist in determining information related to an analyte in a fluid sample, the method comprising the acts of:
providing a base having a first end and a second opposing end;
providing a fluid-receiving area configured to receive a fluid sample;
assigning calibration information to the test sensor; and
wherein the test sensor includes at least one plate thereon such that the size of the at least one plate corresponds to the calibration information, the at least one plate including electrically-conductive material.

### PROCESS D

The method of Process C, wherein the at least one plate is located at or near the second opposing end of the base.

### PROCESS E

The method of Process C, wherein the electrically-conductive material includes silicon carbide, graphite, silver, gold, or platinum.

### PROCESS F

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a potentiometer positioned at or near the test-sensor opening, the potentiometer including a movable actuator, the moveable actuator receiving the at least one notch;
placing the test sensor into the test-sensor opening of the meter;
moving the test sensor so as to move the movable actuator;
determining a measured resistance of the potentiometer; and
applying the calibration information using the measured resistance to assist in determining the information related to the analyte in the fluid sample.

### PROCESS G

The method of Process F, wherein the fluid-receiving area is located at or near the first end.

### PROCESS H

The method of Process F, wherein the at least one notch is formed at or near the second opposing end.

### PROCESS I

The method of Process F, wherein the potentiometer is a plunger-type potentiometer.

### PROCESS J

The method of Process F, wherein the potentiometer is a slide-type potentiometer.

### PROCESS K

The method of Process F further comprising comparing the measured resistance to stored nominal resistance values to determine the calibration information to be applied.

### PROCESS L

The method of Process F further comprising:
removing the test sensor from the test-sensor opening; and
returning the actuator to a default position using a spring mechanism coupled to the actuator.

### PROCESS M

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a pseudo potentiometer positioned at or near the test-sensor opening, the pseudo potentiometer including a plurality of pads, the plurality of pads including resistive materials;
placing the test sensor into the test-sensor opening of the meter;
moving the test sensor so as to cause at least one of the plurality of pads to receive the at least one notch;
determining a measured resistance of the pseudo potentiometer; and
applying the calibration information using the measured resistance to assist in determining the information related to the analyte in the fluid sample.

### PROCESS N

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a variable inductor positioned at or near the test-sensor opening, the variable inductor including a movable plunger and at least one wire coil, the moveable plunger being configured to move within the at least one wire coil, the moveable plunger receiving the at least one notch;
placing the test sensor into the test-sensor opening of the meter;
moving the test sensor so as to move the moveable plunger a distance within the at least one wire coil;
determining a measured electrical value of the variable inductor, the measured electrical value corresponding to the distance that the moveable plunger is moved within the at least one wire coil; and
applying the calibration information using the measured electrical value to assist in determining the information related to the analyte in the fluid sample.

### PROCESS O

The method of Process N, wherein the measured electrical value is an inductance value.

### PROCESS P

The method of Process N, wherein the fluid-receiving area is located at or near the first end.

### PROCESS Q

The method of Process N, wherein the at least one notch is formed at or near the second opposing end.

### PROCESS R

The method of Process N, wherein the moveable plunger is comprised of ferromagnetic materials.

### PROCESS S

The method of Process N, wherein the variable inductor forms a linear variable differential transducer.

### PROCESS T

The method of Process S, wherein the measured electrical value is a voltage value.

### PROCESS U

The method of Process N, wherein the moveable plunger being made from electrically-conductive materials.

### PROCESS V

The method of Process U, wherein the measured electrical value is a frequency value.

### PROCESS W

The method of Process N further comprising comparing the measured electrical value to stored electrical values to determine the calibration information to be applied.

### PROCESS X

The method of Process N further comprising:
removing the test sensor from the test-sensor opening; and
returning the moveable plunger to a default position using a spring mechanism coupled to the moveable plunger.

### PROCESS Y

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a variable capacitor positioned at or near the test-sensor opening, the variable capacitor including a movable plunger and a sleeve, the moveable plunger being configured to move within the sleeve, the moveable plunger receiving the at least one notch;
placing the test sensor into the test-sensor opening of the meter;
moving the test sensor so as to move the moveable plunger a distance within the sleeve;
determining a measured capacitance of the variable capacitor, the measured capacitance corresponding to the distance that the moveable plunger is pushed within the sleeve; and
applying the calibration information using the measured capacitance to assist in determining the information related to the analyte in the fluid sample.

### PROCESS Z

The method of Process Y, wherein the fluid-receiving area is located at or near the first end.

### PROCESS AA

The method of Process Y, wherein the at least one notch is formed at or near the second opposing end.

### PROCESS BB

The method of Process Y, wherein the sleeve is cylindrical.

### PROCESS CC

The method of Process Y further comprising comparing the measured capacitance to stored capacitance values to determine the calibration information to be applied.

### PROCESS DD

The method of Process Y further comprising:
removing the test sensor from the test-sensor opening; and
returning the moveable plunger to a default position using a spring mechanism coupled to the moveable plunger.

### PROCESS EE

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one sensor plate thereon, the at least one sensor plate being made from electrically-conductive materials;
assigning calibration information to the test sensor;
providing a meter with a test-sensor opening, the meter including a meter plate positioned at or near the test-sensor opening, the meter plate being made from electrically-conductive materials;
placing the test sensor into the test-sensor opening of the meter;
moving the test sensor such that at least a portion of the meter plate overlaps at least a portion of the at least one sensor plate;
determining a measured capacitance of the meter plate, the measured capacitance corresponding to the size of the at least one sensor plate; and
applying the calibration information using the measured capacitance to assist in determining the information related to the analyte in the fluid sample.

### PROCESS FF

The method of Process EE, wherein the fluid-receiving area is located at or near the first end.

### PROCESS GG

The method of Process EE, wherein the at least sensor plate is located at or near the second opposing end.

### PROCESS HH

The method of Process EE further comprising comparing the measured capacitance to stored capacitance values to determine the calibration information to be applied.

### PROCESS II

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one sense portion;
assigning calibration information to the test sensor;
providing a meter with a test-sensor opening, the meter including a parallel plate capacitor positioned at or near the test-sensor opening, the parallel plate capacitor including two electrically-conductive plates, the parallel plate capacitor being configured to allow the at least one sense portion of the test sensor to be positioned between the two electrically-conductive plates;
placing the test sensor into the test-sensor opening of the meter;
moving the test sensor such that at least the sense portion of the test sensor is positioned between the two electrically-conductive plates;
determining a measured capacitance of the parallel plate capacitor; and
applying the calibration information using the measured capacitance to assist in determining the information related to the analyte in the fluid sample.

### PROCESS JJ

The method of Process II, wherein the fluid-receiving area is located at or near the first end.

### PROCESS KK

The method of Process II, wherein the at least sense portion is located at or near the second opposing end.

### PROCESS LL

The method of Process II further comprising comparing the measured capacitance to stored capacitance values to determine the calibration information to be applied.

### PROCESS MM

The method of Process II, wherein the measured capacitance corresponds to the thickness of the at least one sense portion.

### PROCESS NN

The method of Process II, wherein the measured capacitance corresponds to the type of material comprising the at least one sense portion.

### PROCESS OO

A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a piezoelectric element positioned at or near the test-sensor opening, the piezoelectric element receiving the at least one notch;
placing the test sensor into the test-sensor opening of the meter;
moving the test sensor so as to compress the piezoelectric element a distance;
determining a measured voltage of the piezoelectric element, the measured voltage corresponding to the distance that the piezoelectric element is compressed; and
applying the calibration information using the measured voltage to assist in determining the information related to the analyte in the fluid sample.

### PROCESS PP

The method of Process OO, wherein the fluid-receiving area is located at or near the first end.

### PROCESS QQ

The method of Process OO, wherein the at least one notch is formed at or near the second opposing end.

### PROCESS RR

The method of Process OO, wherein the piezoelectric element includes quartz or barium titanate.

### PROCESS SS

The method of Process OO further comprising comparing the measured voltage to stored voltage values to determine the calibration information to be applied.

### PROCESS TT

The method of Process OO further comprising:
removing the test sensor from the test-sensor opening; and
allowing the piezoelectric element to return to a default position.

While the invention is susceptible to various modifications and alternative forms, specific embodiments and methods thereof have been shown by way of example in the drawings and are described in detail herein. It should be understood, however, that it is not intended to limit the invention to the particular forms or methods disclosed, but, to the contrary, the intention is to cover all modifications, equivalents, and alternatives falling within the spirit and scope of the invention.

## Claims

1. A method of making a test sensor configured to assist in determining information related to an analyte in a fluid sample, the method comprising the acts of:
providing a base having a first end and a second opposing end;
providing a fluid-receiving area configured to receive a fluid sample;
assigning calibration information to the test sensor; and
forming at least one notch such that a depth of the notch corresponds to the calibration information.

2. A method of making a test sensor configured to assist in determining information related to an analyte in a fluid sample, the method comprising the acts of:
providing a base having a first end and a second opposing end;
providing a fluid-receiving area configured to receive a fluid sample;
assigning calibration information to the test sensor; and
wherein the test sensor includes at least one plate thereon such that the size of the at least one plate corresponds to the calibration information, the at least one plate including electrically-conductive material.

3. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a potentiometer positioned at or near the test-sensor opening, the potentiometer including a movable actuator, the moveable actuator receiving the at least one notch;
placing the test sensor into the test-sensor opening of the meter;
moving the test sensor so as to move the movable actuator;
determining a measured resistance of the potentiometer; and
applying the calibration information using the measured resistance to assist in determining the information related to the analyte in the fluid sample.

4. The method of claim 3, wherein the potentiometer is a plunger-type potentiometer or a slide-type potentiometer.

5. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a pseudo potentiometer positioned at or near the test-sensor opening, the pseudo potentiometer including a plurality of pads, the plurality of pads including resistive materials;
placing the test sensor into the test-sensor opening of the meter;
moving the test sensor so as to cause at least one of the plurality of pads to receive the at least one notch;
determining a measured resistance of the pseudo potentiometer; and
applying the calibration information using the measured resistance to assist in determining the information related to the analyte in the fluid sample.

6. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a variable inductor positioned at or near the test-sensor opening, the variable inductor including a movable plunger and at least one wire coil, the moveable plunger being configured to move within the at least one wire coil, the moveable plunger receiving the at least one notch;
placing the test sensor into the test-sensor opening of the meter;
moving the test sensor so as to move the moveable plunger a distance within the at least one wire coil;
determining a measured electrical value of the variable inductor, the measured electrical value corresponding to the distance that the moveable plunger is moved within the at least one wire coil; and
applying the calibration information using the measured electrical value to assist in determining the information related to the analyte in the fluid sample.

7. The method of claim 6, wherein the measured electrical value is an inductance value, a voltage value, or a frequency value.

8. The method of claim 6 further comprising comparing the measured electrical value to stored electrical values to determine the calibration information to be applied.

9. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a variable capacitor positioned at or near the test-sensor opening, the variable capacitor including a movable plunger and a sleeve, the moveable plunger being configured to move within the sleeve, the moveable plunger receiving the at least one notch;
placing the test sensor into the test-sensor opening of the meter;
moving the test sensor so as to move the moveable plunger a distance within the sleeve;
determining a measured capacitance of the variable capacitor, the measured capacitance corresponding to the distance that the moveable plunger is pushed within the sleeve; and
applying the calibration information using the measured capacitance to assist in determining the information related to the analyte in the fluid sample.

10. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one sensor plate thereon, the at least one sensor plate being made from electrically-conductive materials;
assigning calibration information to the test sensor;
providing a meter with a test-sensor opening, the meter including a meter plate positioned at or near the test-sensor opening, the meter plate being made from electrically-conductive materials;
placing the test sensor into the test-sensor opening of the meter;
moving the test sensor such that at least a portion of the meter plate overlaps at least a portion of the at least one sensor plate;
determining a measured capacitance of the meter plate, the measured capacitance corresponding to the size of the at least one sensor plate; and
applying the calibration information using the measured capacitance to assist in determining the information related to the analyte in the fluid sample.

11. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one sense portion;
assigning calibration information to the test sensor;
providing a meter with a test-sensor opening, the meter including a parallel plate capacitor positioned at or near the test-sensor opening, the parallel plate capacitor including two electrically-conductive plates, the parallel plate capacitor being configured to allow the at least one sense portion of the test sensor to be positioned between the two electrically-conductive plates;
placing the test sensor into the test-sensor opening of the meter;
moving the test sensor such that at least the sense portion of the test sensor is positioned between the two electrically-conductive plates;
determining a measured capacitance of the parallel plate capacitor; and
applying the calibration information using the measured capacitance to assist in determining the information related to the analyte in the fluid sample.

12. The method of claim 11, wherein the measured capacitance corresponds to the thickness of the at least one sense portion or to the type of material comprising the at least one sense portion.

13. The method of claim 9, 10 or 11 further comprising comparing the measured capacitance to stored capacitance values to determine the calibration information to be applied.

14. A method of using a test sensor and a meter, the test sensor and meter using calibration information in determining information related to an analyte in a fluid sample, the method comprising the acts of:
providing a test sensor including a base having a first end and a second opposing end, the test sensor further including a fluid-receiving area configured to receive the fluid sample, the test sensor further including at least one notch formed therein;
assigning calibration information to the test sensor;
providing a meter with a test-sensor opening, the meter including a piezoelectric element positioned at or near the test-sensor opening, the piezoelectric element receiving the at least one notch;
placing the test sensor into the test-sensor opening of the meter;
moving the test sensor so as to compress the piezoelectric element a distance;
determining a measured voltage of the piezoelectric element, the measured voltage corresponding to the distance that the piezoelectric element is compressed; and
applying the calibration information using the measured voltage to assist in determining the information related to the analyte in the fluid sample.

15. The method of claim 3 to 14, wherein the fluid-receiving area is located at or near the first end.
